Europäisches Patentamt

European Patent Office

Office européen des brevets

(11) Publication number: **0 464 233 A1**

(12) **EUROPEAN PATENT APPLICATION**

(21) Application number: **90112553.4**

(22) Date of filing: **02.07.90**

(51) Int. Cl.⁵: **A61K 31/49**, //(A61K31/49, 31:335)

A request for correction of numbering page 13 into 12 has been filed pursuant to Rule 88 EPC. A decision on the request will be taken during the proceedings before the Examining Division (Guidelines for Examination in the EPO, A-V, 2.2).

(43) Date of publication of application:
**08.01.92 Bulletin 92/02**

(84) Designated Contracting States:
**AT BE CH DE DK ES FR GB GR IT LI LU NL SE**

(71) Applicant: **HOECHST AKTIENGESELLSCHAFT**
**Postfach 80 03 20**
**W-6230 Frankfurt am Main 80(DE)**

(72) Inventor: **Chatterjee;Dipak Kumar Dr.**
**Sheetal Bungalow No.2 Mahatma Phule Road**
**Mulund (East)Bombay 400 081(IN)**
Inventor: **Venugopalan;Bindumadhavan Dr.**
**Bldg.45/A,Flat No.31 Brindavan Society**
**Thane 400 061(IN)**
Inventor: **Lal,Bansi Dr.**
**30 A Advani Apartments,Mulund(West)**
**Bombay 400 080(IN)**
Inventor: **De Souza,Noel John Dr.**
**145 Nibbana,Pali Hill,Bandra**
**Bombay 400 050(IN)**
Inventor: **Rupp,Richard Helmut Dr.**
**Roederweg 16a**
**W-6240 Königstein/Taunus(DE)**

(54) Pharmaceutical combinations against malaria.

㊾ Pharmaceutical combinations of Arteether and Quinine or Arteether and Quinine with Mefloquine have an extra ordinary antimalarial activity.

EP 0 464 233 A1

The present invention is concerned with improved antimalarial combinations.

The generic names used here and elsewhere herein are taken from "Tropical Diseases Research, Seventh Programme Report", Chapter 2; Malaria, UNDP/WORLD BANK/WHO, Published by WHO, 1985. The generic name of Quinine is taken from "Acta Leidensia", 55, 21-27, 1987. (E.H.D. Smit, 1987).

Drug-resistant malaria is a serious clinical and public health problem. The malaria parasite Plasmodium falciparum, has developed a versatile capacity of evading the effect of a drug either by genetic manipulation or by nongenetic (adaptive) methods. It has been demonstrated that Chloroquine-resistance in malaria parasites is a stable genetically determined character (D.C. Warhurst (1985). Drug resistance, The Pharmaceutical Journal, No. 23, 689-692). The spread of Plasmodium falciparum resistant to chloroquine and other antimalarial drugs is a major challenge to the health care programme in tropical and subtropical countries (Suphat Noeypatimanond, et al., (1983). Treatment of Plasmodium falciparum malaria with a combination of Amodiaquine and Tetracycline in central Thailand, Trans. R. Soc. Trop. Med. and Hyg. 73 (3), 338-340).

The present combination of antimalarial agents as defined above, and more specifically herein below, permits desirable antimalarial therapy or prophylaxis while preventing or delaying the development of resistance.

In an animal drug, Peters (W. Peters et al. (1977): The Chemotherapy of rodent malaria XXVII. Studies on Mefloquine (WR 142490). Ann. Trop. Med. and Parasit., 71, 407-418) reported that resistance can be slowed down if an antimalarial compound is administered in combination with certain other antimalarial drugs. Peters (W. Peters (1974): Prevention of drug resistance in rodent malaria by the use of drug mixtures. Bull. W.H.O., 51, 379-383); (W. Peters (1984) Drug combination, Handbook of Experimental Pharmacology, Vol. 68/11); Antimalarial drugs (ed. W. Peters and W.H.G. Richards, P.P. Berlin, Heidelberg and New York, Springer-Verlag) also emphasised the use of rational drug combinations for the treatment, but also provide a better therapeutic value. For instance, it has been shown that a triple combination of Mefloquine, Sulfadoxin, and Pyrimethamine delayed resistance development in Plasmodium berghei (B. Merkli, et al., (1980). The inhibitory effect of a drug combination on the development of Mefloquine resistance in Plasmodium berghei. Ann. Trop. Med. and Parasit., 4(1) : 1-9).

The use of combinations of different antimalarials is known in malarial chemotherapy. For example, a combination of Amodiaquine and Tetracycline and a combination of Pyrimethamine and Sulphadoxine known as Fansidar have been used in the clinic (Suphat Noeypatimanond, et al., (1983). Treatment of Plasmodium falciparum malaria with a combination of Amodiaquine and Tetracycline in central Thailand, Trans. R. Soc. Trop. Med. and Hyg. 73 (3), 338-340). Recently, one more antimalarial combination (Fansimef, Mefloquine, Pyrimethamine and Sulphadoxine) is undergoing clinical trials (Tropical Diseases Research, Seventh Programme Report "Chapter 2; Malaria, UNDP/WORLD BANK/WHO, Published by WHO, 1985).

The use of combinations of Artemisinin, its derivatives and other antimalarial compounds, such as Quinine, has been proposed in the Indian Patent Applications 26/BOM/87 and the German Patent Application P 37 15 378. Antimalarial compositions using Quinidine, Artemisinin and its derivatives have been proposed in the Indian Patent-Application 224/BOM/88 and European Patent Application No. 8811 6605.2.

Also the synergistic effect of a combination of Artemisinin and Primaquine is know (Yao-De Wan, Qi-Zhong Zang, Yao Hsueh T'ung Pao, (16(1), 9-12, 14).

It has now been surprisingly found, that a combination of Arteether with Quinine alone or with Quinine + Mefloquine have shown an extraordinary high potentiating effect on oral administration to rodents agains chloroquine-resistant Plasmodium berghei strain.

The subject of the present invention thus is a pharmaceutical combination with a synergistic action against Malaria which, besides customary auxiliaries and/or vehicles contains Arteether and Quinine or Arteether Quinine and Mefloquine.

The surprising new findings have been observed in studies using a four day suppression test with combinations of the invention in Rodent models using Plasmodium berghei NS strain (Ref. Peters W. 1987 Chemotherapy and Drug Resistance in Malaria, Vol. I, P 111).

Experimental data was analysed by the chess-board method in the form of an isobologram which clearly indicates that (1) Arteether in combination with Quinine, offers a strong potentiating effect. These data are described below in further detail.

The antimalarial agent, Arteether, is known. Artheether, is a semi-synthetic derivative of Artemisinin. Its antimalaria' activity is disclosed in different WHO reports. (W.H.O. Report of the Scientific Working Group on the Chemotherapy Malaria, TDR/Chemal 3rd Review, 85. Geneva, 3-5 June 1985 and references contained therein).

The antimalarial agents Quinine and Mefloquine are also known in the literature (W. H. Wernsdorfer (1987) and Sir Ian McGregor "Principles and Practice of Malariology", 1988, Publisher Churchill Livingstone, U.K.).

The clinical value of the present improved formulation in antimalarial therapy is reflected by appropriate animal studies. Typical experimental protocols, in which the ability of the test compound to act as an antimalarial agent was determined even agains drug-resistant strains of P. berghei, are found in the specific examples given below.

The present invention is readily carried out. Arteether is generally dosed in a mammal in the range of 0.125 to 10 mg/kg X 5 days, each day one single dose. The second antimalarial agent Quinine alone or with Mefloquine can be dosed separately, in which case the latter will be employed in an amount within (But generally lower) the dosage range and according to regimens (frequency, routes and compositions) as specified for its utility in the prior art, for example, in the references cited above or further cited in the said references.

Preferably and conveniently, Arteether and the second or third antimalarial agent of the invention are administered in a single, combined formulation. This can be in a form suitable for parenteral administration, but is preferably in a form suitable for oral administration. The proportion of each drug in the proposed combined dosage form will be in the ratio of the total daily dose of each drug when dosed alone. The combined drugs will be dosed in single or divided doses.

In the preferred oral route of dosage, the amount of Arteether for an average adult patient may generally be in the range of 0.2-2.0 g in combination with 750-2000 mg Quinine. The amount administered in the second dose may be administered 6 hours after in the range of 0.02-2 g of Arteether in combination with 250-1000 mg of Quinine for 3 to 6 more days, each day one single dose.

Again a combination of Arteether (range 0.2-1.5 g) together with Quinine (750 mg - 2000 mg) as the first dose may generally be administered to an adult patient. Second dose may be administered 6 hours after the first doese and may contain 0.02-1.5 g of Arteether plus 750-2000 mg of Quinine. The amount administered in the second dose may generally be given for 3 or more days, each day single dose. The above mentioned Quinine doses can also be substituted by a mixture of Quinine and Mefloquine, containing at least 1 % of Quinine.

The combined compounds are administered alone or in further combination with pharmaceutically acceptable carriers or diluents both orally and parenterally. For oral use, suitable pharmaceutical carriers include inert diluents or fillers, thereby forming dosage forms such as tablets, powders, capsules and the like. These pharmaceutical compositions can, if desired, contain additional ingredients such as flavourings, binders, excipients and the like.

For example, tablets containing various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard gelatin capsules, preferred materials therefore include lactose or milk sugar and high molecular weight polyethylene glycols.

For oral formulation the mixture of the compounds can for example be administered in a gelatin capsule. Such formulation could be based on a suitable refined edible oil such as sunflower oil, corn oil, peanut oil, coconut oil or til oil.

The present invention is illustrated by the following examples. It should be however, understood that the invention is not limited to the specific details of the examples.

The strong potentiating effect of Arteether with Quinine against chloroquine sensitive strain of Plasmodium berghei K-173 has been demonstrated by us. The combination of Arteether and Quinine was also tested against chloroquine resistant strain of Plasmodium berghei NS and the results are shown here under in Example 1.

Arteether in combination with Mefloquine also displayed strong potentiating effect against chloroquine resistant Plasmodium berghei NS strain, using the same methodology as shown in Example 1.

## Example 1

Potentiating effect that is synergistic or additive therapeutic effect of sub-curative doses of Arteether in combination with sub-curative doses of Quinine agains Chloroquine-resistant strains of Plasmodium berghei (NS) infected Swiss mice.

The evaluation of blood schizontocidal activities was carried out following the procedure described herein after using the Chloroquine-resistant strain of P. berghei (NS) strain. (Chawira A. N., et al. 1987, Trans, R. Soc. Trop. Med. Hyg. 81 : 554-558).

The methodology followed in the present study was adopted form "4- day suppressive test" (ref. Peters, W.; 1987, Chemotherapy and Drug Resistance in Malaria, Vol. 1, p 111). The experimental data has been analysed by Chess Board Method in the form of an isobologram, (Ref. Chawira A. N., and Warhurst D. C.; 1987, J. Trop. Med. Hyg. 90 : 1-8). $ED_{50}$ and $ED_{90}$ was determined using a log-probit method.

## General Methodology

| | |
|---|---|
| Host | .. Swiss Mice (20-22 gms) |
| Sex | .. Male |
| Parasit | .. P. berghei NS (resistant to chlorquine) |
| Route of Infection | .. Intravenous |
| Route of drug Administration | .. per os |

## Preparation of the drug

Artheether : A few drops of Kardi oil and Tween[R] 80 were added to Arteether to make a paste, followed by 0.5 % Tylose[R] (Carboxy methyl cellulose) solution. A homogenous suspension was prepared using a tissue homogenizer in Teflon[R] coated glass tube.

Quinine sulphate : A solution was prepared in 0.5 % Tylose using a tissue homogenizer.

Mefloquine HCl : A solution was prepared in 0.5 % Tylose using a tissue homogenizer.

Arteether was drenched using stomach tube (0.5 ml) followed by either Quinine (0.5 ml) or Mefloquine (0.5 ml) as required.

Drug was administered as follows:

(+ 2 H, 24 H, 48 H, 72 H)

Each day one dose for 4 days

## Blood slides

## Control - Infected untreated

Slides were drawn from individual mouse and stained with Giemsa on D + 4. The slides were examined under oil immersion lens and parasitaemia was recorded from 15 fields at random. The average of infected and uninfected erythrocytes was determined for each animal. Finally the average parasitaemia of the whole group was determined and considered as 100 % for comparison purposes with the treated group.

## Parasitaemia of the treated group

At least 5000 to 7500 erythrocytes were examined at random to determine the parasitaemia. In certain cases, where parasitaemia was extremely low 10000 to 15000 erythrocytes were examined.

## Results

## Arteether and Quinine Combination

The result of the above combination therapy is presented in Table 1.

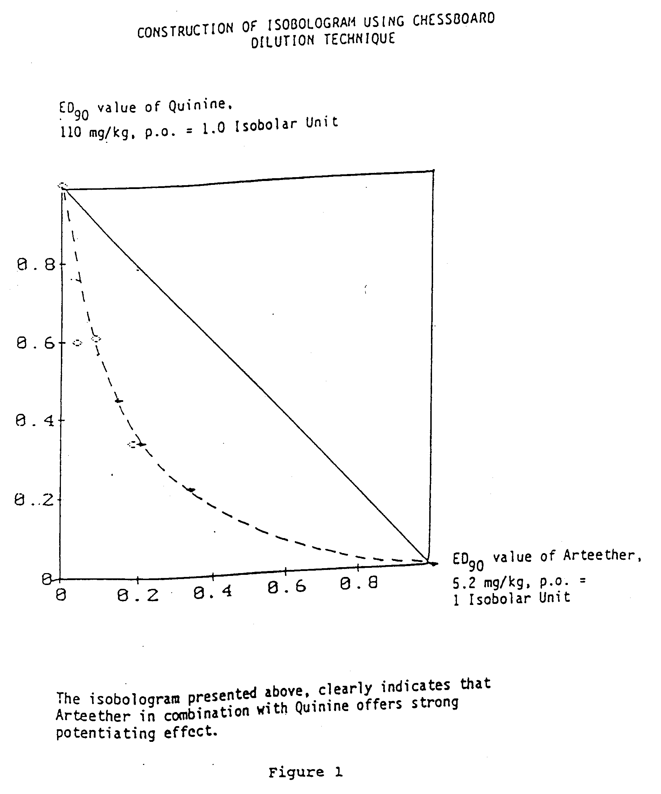

$ED_{50}$ and $ED_{90}$ values of Arteether have been calculated by extending the doses of the compound in the same experiment (Table 2). The experimental data of the above study have been converted in the isobologram in Figure 1.

## Remarks

The isobologram presented in Figure 1 clearly indicates that Arteether in combination with Quinine offers strong potentiating effect.

NS strain has some unique biological features in resembling P. falciparum and has moderate resistance to chloroquine.

## Table 1

Arteether and Quinine Combination Agaings P. berghei, WS
Strain in Male Mice

| Quinine mg/kg X 4, p.o. | Arteether mg/kg X 4, p.o. | | | | | |
|---|---|---|---|---|---|---|
| | 0.25 | 0.5 | 1.0 | 2.0 | $ED_{50}$ | $ED_{90}$ |
| 100 | Control<br>$\pm$ 5.04 | 90<br>$\pm$ 13.9 | 87<br>$\pm$ 10.8 | 84<br>$\pm$ 10.8 | 77<br>$\pm$ 1.27 | 2.8<br>(2.1-3.6) |
| | | | | | | 5.2<br>(4.0-6.7) |
| 25 | 92<br>$\pm$ 2.36 | 89<br>$\pm$ 1.86 | 33<br>$\pm$ 5.85 | 33<br>$\pm$ 5.85 | 8.9<br>$\pm$ 2.25 | 0.68<br>(0.61-0.75) |
| | | | | | | 1.8<br>(1.29-2.3) |
| 37.5 | 85<br>$\pm$ 5.8 | 53<br>$\pm$ 5.5 | 43<br>$\pm$ 5.88 | 11<br>$\pm$ 2.47 | 0.8<br>$\pm$ 0.25 | 0.33<br>(0.2-0.54) |
| | | | | | | 1.1<br>(.66-1.8) |
| 50 | 70<br>$\pm$ 11.8 | 36.5<br>$\pm$ 8.73 | 24<br>$\pm$ 2.27 | 0.8<br>$\pm$ 0.66 | 0.13 | |
| 75 | 23<br>$\pm$ 10.9 | 5.84<br>$\pm$ 3.08 | 51<br>$\pm$ 1.95 | 0.73<br>$\pm$ 0.42 | 0* | |
| $ED_{50}$ | 61<br>(43.33-85.84) | 40<br>(33.6-47.5) | 34<br>(29.5-39.1) | 0* | 0* | |
| $ED_{90}$ | 110<br>(78.1-154.8) | 66<br>(55.5-78.3) | 68<br>(59.0-78.3) | 0* | 0* | |

The Table shows group mean of parasitaemia as of control, standard error,
$ED_{50}$ and $ED_{90}$ values (lines fitted by eye).

EP 0 464 233 A1

Table II

$ED_{50}$ and $ED_{90}$ values of Arteether Against P. berhei NS in Male Mouse

| Dose mg/kg X 4 | X Parasitaemia | $ED_{50}$ | $ED_{90}$ |
|---|---|---|---|
| 2.0 | 82 | 2.8 | 5.2 |
| 3.0 | 57 | (2.1-3.6) | (4.0-6.7) |
| 4.0 | 25 | | |
| 5.0 | 12 | | |

## Claims

1. A pharmaceutical combination with a synergistic action against malaria, which besides customary auxiliaries and/or vehicles contains Arteether and Quinine or Arteether and Quinine with Mefloquine.

2. A pharmaceutical as claimed in claim 1, which contains Arteether and Quinine.

3. A pharmaceutical as claimed in claim 1, which contains Arteether and Quinine with Mefloquine.

4. A pharmaceutical as claimed in one or more of claims 1 - 3, containing 0.2 - 2 g Arteether in combination with 750 - 2000 mg Quinine or mixture of Quinine with Mefloquine, containing at least 1 % Quinine.

5. A pharmaceutical as claimed in one or more of claims 1 - 3, containing 0.02 - 2 g Arteether in combination with 250 - 1000 mg Quinine or mixture of Quinine with Mefloquine containing at least 1 % Quinine.

6. The use of a combination of compounds as claimed in one or more of claims 1 - 5 for the preparation of pharmaceuticals with a synergistic action against malaria.

7. A method of treatment or prophylaxis of malaria, wherein a pharmaceutical combination as claimed in one or more of claims 1 - 5 is administered.

CONSTRUCTION OF ISOBOLOGRAM USING CHESSBOARD
DILUTION TECHNIQUE

$ED_{90}$ value of Quinine.
110 mg/kg, p.o. = 1.0 Isobolar Unit

$ED_{90}$ value of Arteether,
5.2 mg/kg, p.o. =
1 Isobolar Unit

The isobologram presented above, clearly indicates that Arteether in combination with Quinine offers strong potentiating effect.

Figure 1

7

European Patent
Office

**PARTIAL EUROPEAN SEARCH REPORT**
which under Rule 45 of the European Patent Convention
shall be considered, for the purposes of subsequent
proceedings, as the European search report

Application number

EP 90 11 2553

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 290 959 (HOECHST AG) <br><br> * Page 10, line 1 - page 11, line 20; claims 1-26 * <br><br> ---- | <br><br>1-6 | A 61 K 31/49// (A 61 K 31/49 A 61 K 31:335) |

**TECHNICAL FIELDS SEARCHED (Int. Cl.4)**

A 61 K

## INCOMPLETE SEARCH

The Search Division considers that the present European patent application does not comply with
the provisions of the European Patent Convention to such an extent that it is not possible to carry
out a meaningful search into the state of the art on the basis of some of the claims.

Claims searched completely: 1-6

Claims searched incompletely:

Claims not searched: 7

Reason for the limitation of the search:

Method for treatment of the human
or animal body by surgery or therapy
(See art. 52(4) of the European
Patent Convention)

| Place of search | Date of completion of the search | Examiner |
|---|---|---|
| THE HAGUE | 21-02-1991 | BRINKMANN |

EPO Form 1505.1 03.82